Europäisches Patentamt

European Patent Office

Office européen des brevets

(11) Veröffentlichungsnummer: **0 388 696**

**A1**

(12) **EUROPÄISCHE PATENTANMELDUNG**

(21) Anmeldenummer: **90104172.3**

(22) Anmeldetag: **03.03.90**

(51) Int. Cl.⁵: **B65D 47/32, A61M 5/14**

(30) Priorität: **22.03.89 DE 8903605 U**

(43) Veröffentlichungstag der Anmeldung:
**26.09.90 Patentblatt 90/39**

(84) Benannte Vertragsstaaten:
**AT BE CH DE DK ES FR GB GR IT LI LU NL SE**

(71) Anmelder: **Fresenius AG**
**Gluckensteinweg 5**
**D-6380 Bad Homburg v.d.H.(DE)**

(72) Erfinder: **Ufermann, Rüdiger**
**Scherpenberger Strasse 111**
**D-4130 Moers 1(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**D-6200 Wiesbaden(DE)**

(54) **Überleitgerät für Flaschen, die mit einer medizinisch wirksamen Flüssigkeit ausgefüllt sind.**

(57) Es wird ein Überleitungsgerät für Flaschen beschrieben, die eine enteral zu verabreichende Flüssigkeit aufweisen. Das Überleitgerät weist ein Steigrohr (11) auf, dessen Ende mit einem hydrophoben Filter (12) verschlossen ist.

Fig. 2

## Überleitgerät für Flaschen, die mit einer medizinisch wirksamen Flüssigkeit angefüllt sind

Die Neuerung bezieht sich auf ein Überleitgerät für Flaschen, die mit einer medizinisch wirksamen Flüssigkeit angefüllt sind, die in "Über-Kopf-Stellung" der Flasche tröpfelnd entleert wird, mit einem der Flaschenöffnung angepaßten Verschlußteil, das mit einem Schlauch für die Applikation der Flüssigkeit in Verbindung steht und das einen verschließbaren Belüftungskanal aufweist.

Ein derartiges Überleitgerät ist u.a. durch Verkauf, Ausstellung bzw. Anwendung einschlägiger Produkte bekannt geworden.

Überleitgeräte dieser Art werden im Anwendungsfall mit ihrem Verschlußteil z.B. auf eine Kronenkorken-Flasche mit Nährflüssigkeit bei der Sondenernährung, aufgedrückt. Bei diesem Aufdrücken kommt es häufig zum Einlaufen von Flüssigkeit in den Belüftungskanal, selbst dann, wenn dieser ein Steigrohr aufweist, das tief in die Flasche eintaucht.

Dies kann dazu führen, daß keine Belüftung mehr erfolgt und der Flüssigkeitsfluß zum Erliegen kommt. Ferner tritt die Flüssigkeit, die klebrig sein kann, dann sehr oft an der der Umgebung zugewandten Öffnung des Belüftungskanals aus. Dies ist beim Umgang mit dem Überleitgerät und den Flaschen sehr störend und auch unhygienisch.

Der Neuerung liegt die Aufgabe zugrunde, ausgehend von dem eingangs bezeichneten Übrleitgerät dieses so auszubilden, daß jederzeit eine Belüftung gewährleistet ist, ohne das Flüssigkeit durch den Belüftungskanal austritt.

Die Lösung dieser Aufgabe gelingt neuerungsgemäß dadurch, daß die flaschenseitig zugewandte Öffnung des Belüftungskanals mit einem hydrophoben Filter verschlossen ist.

Bei gemäß der Neuerung mit einem hydrophoben Filter ausgestatteten Überleitgeräten kann auch bei mehrfachem Wechsel des Überleigerätes auf andere Flaschen keine Flüssigkeit mehr in den Belüftungskanal gelangen. Die Belüftung ist daher jederzeit gewährleistet und Flüssigkeit kann nicht in die Umgebung austreten.

Das Verschließen von Belüftungskanälen an mit Flüssigkeit gefüllten Flaschen durch hydrophobe Filter ist an sich aus der Infusionstechnik bekannt. Im bekannten Fall ist jedoch die andere, die der Umgebung zugewandte Öffnung des Belüfungskanals mit dem Filter verschlossen, um ein Eindringen von Fremdkörpern von aussen in das System zu verhindern. An ein Verhindern des Eindringens der Flüssigkeit in den Belüftungskanal aus der Flasche heraus d.h. von innen, ist im bekannten Fall nicht gedacht. Die Fachwelt war bestrebt, das unmittelbare Benetzen des Filters mit der Flüssigkeit zu vermeiden. Obwohl im Fall der Neuerung das Filter beim Aufsetzen des Verschlußteiles direkt mit der Flüssigkeit in Kontakt kommt, bleibt, wie Versuche gezeigt haben, das Filter voll wirksam.

Gemäß einer Weiterbildung der Erfindung weist der Belüftungskanal flaschenseitig ein Steigrohr auf, dessen freies Ende mit dem hydrophoben Filter versehen ist. Durch dieses "Hochlegen" des hydrophoben Abschlußfilters (in der Gebrauchslage "Über-Kopf") ist die zur einwandfreien Funktion notwendige Differenz zwischen Lufteintritt und Flüssigkeitsaustritt (die möglichst groß sein sollte) in jedem Fall sichergestellt.

Weitere ausgestaltende Merkmale und Anwendungsmöglichkeiten der Erfindung ergeben sich anhand der Beschreibung von in den Zeichnungen dargestellten Ausführungsbeispielen.

Es zeigen:

Figur 1 ein Überleitgerät, dessen Verschlußteil einen Belüftungskanal mit anschließendem Steigrohr mit aufgesetztem hydrophoben Filter aufweist,

Figur 1a einen Ausschnitt aus Figur 1 mit einer anderen Ausführungsform der Anordnung des Filters.

Figur 1b einen weiteren Ausschnitt aus Figur 1 mit einer weiteren Ausführungsform der Anbringung des hydrophoben Filters.

Figur 2 einen Ausschnitt aus einem Überleitgerät mit einem Verschlußteil, bei dem das hydrophobe Filter ohne Steigrohr direkt auf ein Entlüftungsrohr im Verschlußteil aufgesteckt ist.

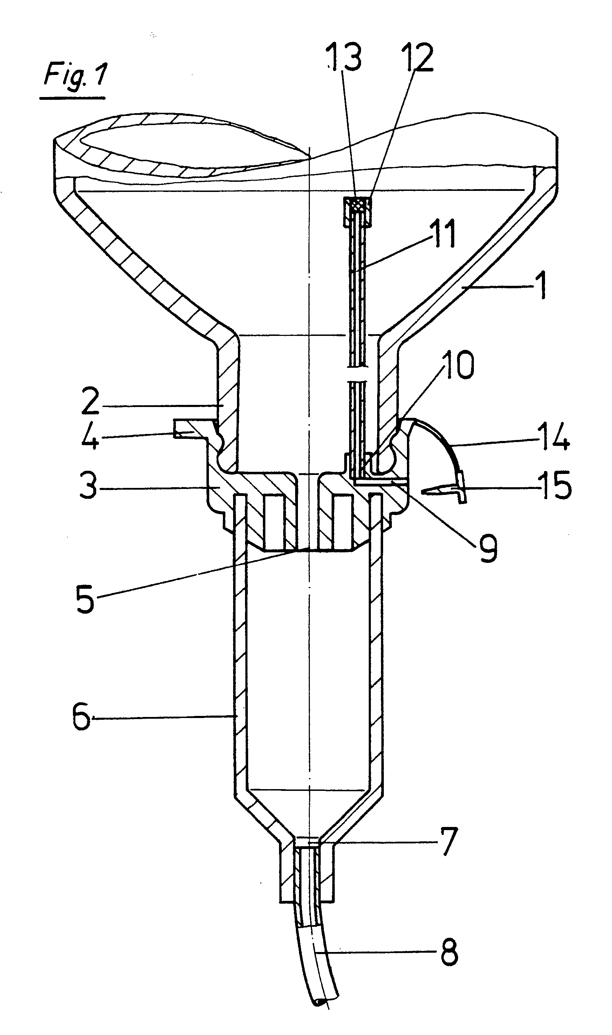

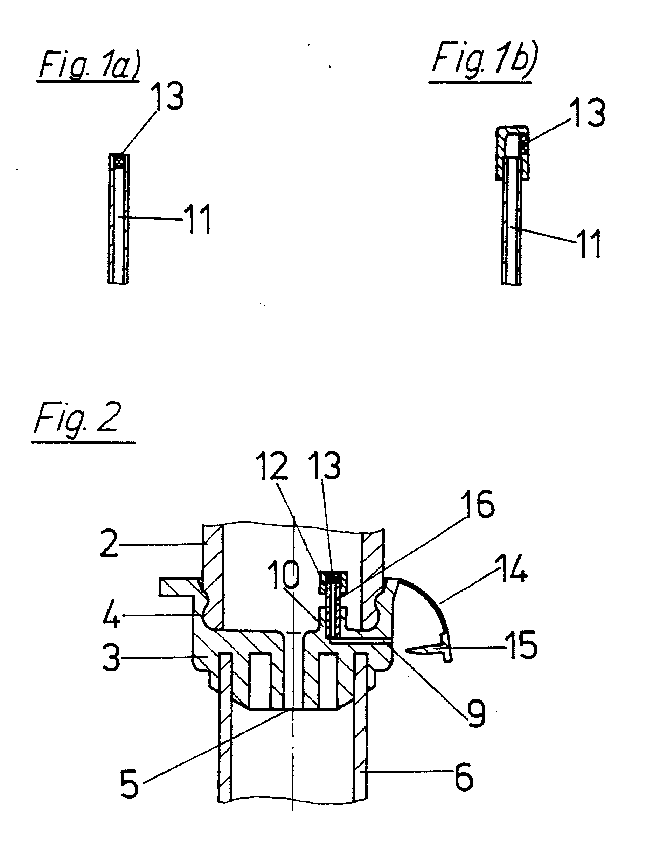

Die Figur 1 zeigt die wesentlichen Teile eines Überleitgerätes für eine Flasche 1, die mit einer medizinisch wirksamen Flüssigkeit angefüllt ist, die in "Über-Kopf-Stellung" der Flasche tröpfelnd entleert wird.

Das Überleitgerät weist ein der Flaschenöffnung angepaßtes Verschlußteil 3 mit einem Ansatz 4 für den Flaschenrand 2 und einer Durchtrittsöffnung 5 für die Flüssigkeit auf. Das Verschlußteil ist integral mit einer Tropfkammer 6 verbunden, an deren Ausgang 7 ein Schlauch 8 für die Applikation der Flüssigkeit angebracht ist. Die weitere Ausbildung dieses Applikationsschlauches 8 ist bekannt und braucht hier nicht näher dargestellt zu werden.

Zur Belüftung des Flascheninneren beim Entleeren der Flüssigkeit weist das Überleitgerät eine Belüftungsbohrung 9 auf, die im Ansatz 4 in Form eines kleinen Fortsatzes 10 ausläuft. In diesem Fortsatz ist ein Steigrohr 11 angebracht, dessen oberes Ende mit einem hydrophoben Filter 12 verschlossen ist. In der Ausführungsform nach Figur 1 ist das Filter 12 auf das Steigrohr 11 aufgesteckt. Das Filter 12 besteht dabei aus einem Filtergehäuse mit einem Filtereinsatz 13, dessen Durchlaßrichtung koaxial mit der Steigrohrachse verläuft.

Die Belüftungsbohrung 9 kann in einer ersten Ausführungsform an der Lufteinrittsseite zweckmäßig verschließbar ausgebildet sein, z.B. in Form einer herunterhängenden Verschlußkappe 14 mit einem der Belüftungsbohrung angepaßten Dorn 15.

In einer zweiten Ausführungsform bleibt die Belüftungsbohrung 9 permanent offen, da die Verschlußkappe 14 mit Dorn 15 nicht mehr vorgesehen ist. Letztere Ausführung ist dabei aus spritzgußtechnichen Gründen bevorzugt.

Nach einer entsprechenden Ausgestaltung der Erfindung gemäß Figur 1a kann der Filtereinsatz 13 auch unmittelbar in das obere Ende des Steigrohres 11 eingedrückt werden, so daß die Notwendigkeit eines Filtergehäuses entfällt.

Gemäß einer weiteren Ausgestaltung der Erfindung entsprechend Figur 1b ist der Filtereinsatz 13 peripher an einem Filtergehäuse in Form einer Verschlußkappe mit seiner Durchlaßrichtung senkrecht zur Steigrohrachse angebracht. Leztere Ausführungsform nach Figur 1b ermöglicht ein sicheres Ablaufen unerwünschter Flüssigkeit auf dem Filter nach dem Eintauchen des Steigrohres in den Flascheninhalt.

In Figur 2 ist eine weitere Ausführungsform eines Verschlußteiles eines Überleitgerätes dargestellt, wobei mit der Ausführungsform nach Figur 1 identische Teile mit denselben Bezugsziffern versehen sind.

Bei der Ausführungsform nach Figur 2 ist das hydrophobe Filter 12 unmittelbar, d.h. ohne Steigrohr an dem Verschlußteil 3 angebracht. Dies könnte prinzipiell geschehen, indem man das Filter 12 unmittelbar mit dem Fortsatz 10 verbinden würde. Konstruktiv günstiger ist es, zur Verlängerung der Belüftungsbohrung 9 ein Belüftungsröhrchen 16 vorzusehen, z.B. ein kleines Kunststoffröhrchen, auf welches dann das Filter 12 aufgesteckt ist. Der Filtereinsatz 13 kann auch in das Belüftungsröhrchen 16 eingeschoben werden (entsprechend Figur 1a). Auch ist eine Filterbefestigung entsprechend der Ausführungsform nach Figur 1b grundsätzlich denkbar.

Die Filter werden bei den Ausführungsformen nach den Figuren 1 und 2 vorzugsweise so aufgesteckt, daß der Filtereinsatz 13 bündig mit der Austrittsöffnung des Steigrohres 11 bzw. des Belüftungsröhrchens 16 ist. Es ist auch grundsätzlich möglich, den Filtereinsatz etwas geneigt dazu anzubringen.

Auch ist grundsätzlich eine Integralbauweise möglich, d.h., das Belüftungsröhrchen 16 der Figur 2 könnte als hydrophobes rohrförmiges Teil mit einem integrierten Filtereinsatz ausgebildet sein.

Bei der Ausführungsform nach Figur 2 müssen die Luftblasen den Weg durch die Flüssigkeit gehen. Bei höherviskosen Flüssigkeiten ist dies nicht immer sichergestellt. Daher wird die Ausführungsform nach Figur 2 in solchen Fällen zweckmäßig bei pumpenbetriebenen Überleitgeräten eingesetzt.

Als Filtermaterialien kommen vorzugsweise die üblichen Kunststoffmaterialien in Frage, die für medizinische Zwecke bzw. für Nahrungsmittel zugelassen sind.Einsetzbar sind hydrophobe mikroporöse Membranen, die vorteilhafterweise zum Belüften von Infusionslösungen eingesetzt werden und in Infusionsüberleitgeräten vorgesehen sind. Diese Membranen lassen Luft unter sterilen Bedingungen durchtreten und weisen üblicherweise eine mittlere Porengröße von 0,2-0,5/um auf.

Die Erfindung findet vorzugsweise Verwendung als Überleitgerät für Flaschen mit Nahrungsmitteln zur Sondenernährung. Sie kann jedoch auch entsprechend bei Infusionsgeräten oder bei zu belüftenden Medikamentenfläschchen eingesetzt werden.

## Ansprüche

1 Überleitgerät für Flaschen (1), die mit einer medizinisch wirksamen Flüssigkeit angefüllt sind, die in "Über-Kopf-Stellung" der Flasche tröpfelnd entleert wird, mit einem der Flaschenöffnung angepaßten Verschlußteil (3), das mit einem Schlauch (8) für die Applikation der Flüssigkeit in Verbindung steht und das einen verschließbaren Belüftungskanal aufweist, dadurch gekennzeichnet, daß die der Flaschenseite zugewandte Öffnung des Belüftungskanals (9,11,9,16) mit einem hydrophoben Filter (12) verschlossen ist.

2. Überleitgerät nach Anspruch 1, dadurch gekennzeichnet, daß der Belüftungskanal flaschenseitig ein Steigrohr (11) aufweist.

3 Überleitgerät nach Anspruch 1, dadurch gekennzeichnet, daß der Belüftungskanal flaschenseitig einen Stutzen (10,16) aufweist.

4. Überleitgerät nach Anspruch 1 oder 2, 3, dadurch gekennzeichnet, daß das Filter (12) auf das Steigrohr (11) bzw. auf den Stutzen (10,16) aufgesteckt ist.

5. Überleitgerät nach Anspruch 1 oder 2, 3, dadurch gekennzeichnet, daß das Filter (12) in das Steigrohr (11) bzw. in den Stutzen (10,16) eingedrückt ist.

6. Überleitgerät nach Anspruch 4, dadurch gekennzeichnet, daß das Filter mit seinem Einsatz (13) bündig mit der Austrittsöffnung des Steigrohres (11) bzw. des Stutzens (10,16) oder geneigt dazu, mit seiner Durchlaßrichtung koaxial mit dem Steigrohr bzw. dem Stutzen angeordnet ist.

7. Überleitgerät nach Anspruch 4, dadurch gekennzeichnet, daß der Einsatz (13) des Filters (12)peripher an einer Art Verschlußkappe mit seiner Durchlaßrichtung senkrecht zur Steigrohrachse angebracht ist.

8. Überleitgerät nach einem der Ansprüche 1-7, dadurch gekennzeichnet, daß das Filter aus Kunststoffmaterialien besteht.

9. überleitgerät nach einem der Ansprüche 1-8, gekennzeichnet durch die Verwendung für Flaschen mit flüssigen Nahrungsmitteln für eine Sonderernährung.

10. Überleitgerät nach einem der Ansprüche 1-8, gekennzeichnet durch die Verwendung für Flaschen mit Infusionslösungen.

11. Überleitgerät nach einem der Ansprüche 1-8 gekennzeichnet durch die Verwendung bei zu belüftenden Medikamentenfläschchen.

Fig. 1

Fig. 1a)

Fig. 1b)

Fig. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| X | <u>GB - A - 2 063 836</u><br>(BAXTER TRAVENOL LABORATORIES INC.)<br>* Gesamt; insbesondere Seite 1, Zeilen 112-128 *<br>-- | 1,2,4,<br>8 | B 65 D 47/32<br>A 61 M 5/14 |
| A | <u>DE - A1 - 2 853 946</u><br>(TRANSCOOLAN SREN HUSTED-AN-DERSEN)<br>* Gesamt; insbesondere Fig. 2,3 *<br>-- | 1,2 | |
| A | <u>US - A - 3 359 977</u><br>(BURKE)<br>* Fig. 1,3 *<br>---- | 1 | |

RECHERCHIERTE SACHGEBIETE (Int. Cl⁵)

B 65 D 47/00
B 65 D 51/00
A 61 M  5/00
A 61 M 39/00

Der vorliegende Recherchenbericht wurde für alle Patentansprüche erstellt.

| Recherchenort<br>WIEN | Abschlußdatum der Recherche<br>28-06-1990 | Prüfer<br>CZUBA |
|---|---|---|